# EUROPEAN PATENT SPECIFICATION

(11) **EP 3 610 906 B1**
(45) Date of publication and mention of the grant of the patent: **04.11.2026**
(21) Application number: 19201260.7
(22) Date of filing: 26.01.2016
(51) Int. Cl.: A61M 5/168, A61M 5/142, G01N 33/15

(54) **SYSTEM FOR DOSE RATE REGULATION IN DRUG INFUSION TREATMENT**
SYSTEM ZUR DOSISRATENREGULIERUNG IN DER MEDIKAMENTENINFUSIONSBEHANDLUNG
SYSTÈME DE RÉGULATION DE DÉBIT DE DOSE DANS UN TRAITEMENT DE PERFUSION DE MÉDICAMENT

(30) Priority: 27.01.2015 SE 1550082
(43) Date of publication of application: 19.02.2020
(62) Document number(s) of the earlier application(s) in accordance with Art. 76 EPC: 16743792.0 / 3 250 259
(73) Proprietor: RaySearch Laboratories AB, 104 30 Stockholm (SE)
(72) Inventor: DAHLIN, Hans, 743 63 Björklinge (SE); HÖJDMO, Jörgen, 756 45 Uppsala (SE)

(56) References cited:
- WO-A1-2004/109262
- ES-T3- 2 249 665
- KR-A- 20130 125 086
- US-A1- 2003 204 330
- US-A1- 2010 305 499

## Description

### FIELD OF THE INVENTION

The present invention relates to the administration of drugs by infusion, and more particularly to a system for infusion of a drug-containing liquid involving fluid analysis of the identity and concentration of the drug or drugs that are to be administered.

### BACKGROUND OF THE INVENTION

Delivery of the correct medication, dose and volume at the appropriate infusion rate and time is essential for safe administration of medication by infusion, generally intravenously.

Drug solutions for administration by infusion are provided in various ways. Certain drug solutions, such as cytostatic drugs, are prepared at hospital pharmacies or at drug manufacturers, whereas other drugs, such as antibiotics and morphine, are prepared at hospital pharmacies or at the wards, either from a base solution or a powder formulation.

While the hospital routines are rigorous, the risk for mistakes by pharmacy and hospital personnel cannot be totally avoided. Not only can mistakes be made in the manual preparation of a drug solution, but for example mix-up of different medicaments may also take place at the moment when the drug container is connected to the device used for the administration. Since drugs for infusion are often highly potent, errors in drug composition and concentration may have serious effects on the patient and can even be fatal.

To eliminate the consequences of such mistakes devices have been proposed which immediately before infusion analysis whether the administration container set up for infusion contains the correct drug or not, and if the concentration of the drug solution is as prescribed.

WO 2004/109262 A1 discloses a method and a device to perform verification of the identity of a drug in an administration container by loading drug from the container to an analyzing unit, non-invasively determining a value of at least one chemical and/or physical property of a drug solution to generate a profile for the drug, and comparing the obtained profile with a set of known profiles. If agreement between the obtained profile and the profile of the prescribed drug is reached a message is issued that the treatment is safe and administration of the drug can proceed. Optionally, also the concentration of the drug is verified before the administration is allowed to proceed. The non-invasive analysis is preferably performed by absorption spectrophotometry.

A similar approach is disclosed in WO 2004/033003 A1, where a device and process for preventing medical errors due to the improper administration of an intravenously delivered medication include spectroscopic analysis of intravenous fluid components. An emission source and a detector are placed adjacent to the intravenous tubing of an administration set to generate signals for spectroscopic analysis. The signals are processed to identify the medication and preferably also the concentration of the medication. In a preferred embodiment, the emission source, detector and hardware and software for the spectroscopic analysis are placed in an infusion pump.

In US2003/0204330 a device and process for preventing medical errors due to the improper administration of an intravenously delivered medication includes the spectroscopic analysis of intravenous fluid components. US 2010/305499 A1 discloses a further prior art approach.

In the devices and processes outlined above, failure to verify the identity of the drug in the drug administration container may result in disposal of the container and replacement with a new drug container. Disposal of the drug container would also be the result if the concentration of the drug is determined and is found not to correspond to or to unacceptably deviate from the label concentration.

It is an object of the present invention to provide means for proceeding with the administration of the drug solution even if the determined concentration is not the correct one or is within an acceptable range.

As is well known, drug molecules may adhere to the inner wall of the drug container, such as an infusion bag, which may result in an inhomogeneous drug solution within the container which in turn may result in a varying drug concentration of the drug solution administered to the patient, even if the container has been gently shaken or massaged before the set up. A varying drug concentration may also be due the drug solution undergoing a change during the infusion process.

It is another object of the present invention to deliver a correct dosage of a drug administered by infusion even if the concentration of the administered drug solution varies during the infusion.

It is another object of the present invention to provide a method and a system capable of identifying the drug and the concentration by means of spectrophotometry.

### SUMMARY OF THE INVENTION

According to the present invention, which is defined in the independent claim 1, the above-mentioned objects and other advantages are obtained by providing a drug delivery system where the concentration of the drug solution withdrawn from the drug container to be delivered to the patient according to a predetermined (prescribed) dose rate protocol for the drug is repeatedly analyzed during the infusion, and the concentration data obtained are used to continuously control the infusion rate to at least substantially maintain the predetermined dose rate.

In another aspect, there is provided a system for administering a drug solution by infusion, comprising
a drug container containing a label drug solution with a label concentration of a drug,
a drug delivery set comprising a fluid conduit connected to the drug container, means for connecting the fluid conduit to a patient, a detection chamber in the fluid conduit between the drug container and the patient connection means, and adjustment means for setting an infusion rate corresponding to a predetermined dose rate of the drug based on the label concentration,
analytical detection means for non-invasive determination in the detection chamber of the identity and concentration of the drug in fluid withdrawn from the drug container into to the fluid conduit, and
means for preventing infusion of drug solution from the drug container if the label drug is not identified, and
a control unit arranged to control the adjustment means and configured to calculate and set the adjustment means to an adjusted infusion rate in order to maintain the predetermined dose rate when the drug concentration determined in the detection chamber deviates from the label concentration of the drug,
wherein said system further comprises a blood analysis system to measure the biological response to the actual dose rate, wherein information obtained therefrom is used as a variable in the dose rate setting.

The terms "label drug" and "label concentration" as used herein are to be interpreted in a broad sense and do not necessarily refer a label on the drug container but also includes information on the identity and concentration, respectively, of the specific drug provided in any other way.

The terms "predetermined infusion rate" and "predetermined dose rate" as used herein refer to a predetermined infusion rate and dose rate, respectively, which can either be fixed or varied in a predetermined manner.

In one embodiment of the method and the system, the determination of the drug concentration is performed repeatedly at a plurality of times during the infusion process, an adjusted infusion rate being calculated and set each time based on the momentarily determined concentration as well as the cumulated infused dose of the drug. In this way, the desired (prescribed) total dose of the drug will be administered if the infusion is performed during the infusion time length corresponding to that set for the label drug concentration and the desired dose rate of the drug.

In one embodiment, the drug delivery set comprises an infusion pump, typically a peristaltic pump or a syringe pump, which comprises the adjustment means controlled by the control unit.

In one embodiment, the detection chamber, and optionally also the analytical detection means and (partially or wholly) the control unit, are provided in the infusion pump.

In one embodiment, the predetermined dose rate of the drug is varied in a predetermined manner with different fixed dose rates delivered at different intervals, such as, for example, delivering a higher dose rate for an initial period of the infusion time, and a lower dose rate for the remaining infusion time, or vice versa.

A more complete understanding of the invention, as well as further features and advantages thereof, will be obtained by reference to the following detailed description and drawing. Further embodiments are also disclosed in the patent claims.

### BRIEF DESCRIPTION OF THE DRAWINGS

Figure 1 is a schematic view of an embodiment of a system for drug infusion
Figure 2 is a schematic view of an embodiment of an analytical detection unit.
Figure 3 is a flow diagram illustrating an embodiment of a method for drug infusion

### DETAILED DESCRIPTION OF THE INVENTION

The following description is for illustration and exemplification of the invention only and is not intended to limit the invention to the specific embodiments described. Unless defined otherwise, technical and scientific terms have the same meaning as commonly understood by one of ordinary skill in the art to which this invention belongs. The meanings of the terms dose, infusion rate, dose rate, and spectrophotometric curves as used herein are as follows.

The "dose" of a drug is the amount of drug intended for infusion over a specific length of time, e.g. in mg.

The "infusion rate" (or flow rate) is the liquid volume infused per time unit, e.g. mL/minute, determining how rapidly the infusion is delivered to the patient.

The "dose rate" is the amount of drug delivered per time unit, e.g. mg per minute, and is determined by the concentration of the drug and the infusion rate.

The "spectrophotometric curve" is a set of data points, wherein each data point is a spectrophotometric variable at a specific wavelength of light. For example in a preferred embodiment the spectrophotometric parameter is the absorbance of light. But other spectrophotometric variable such as reflectance is of course also possible.

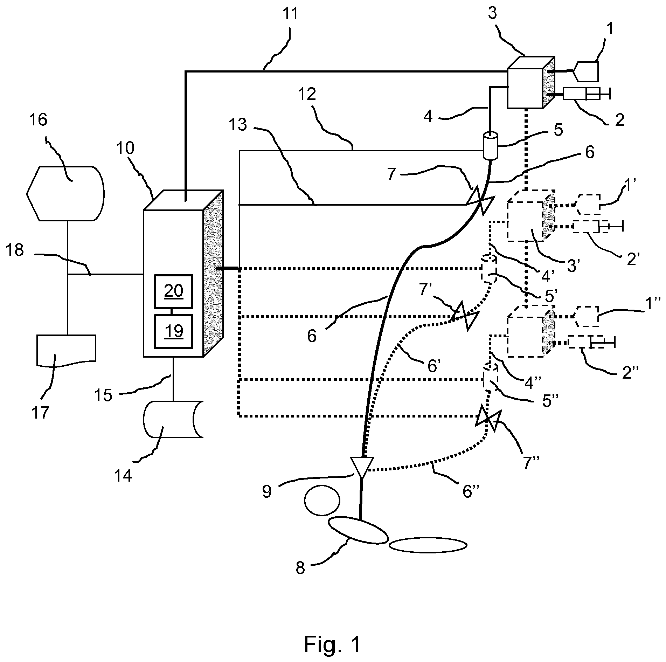

As mentioned above, the present invention relates to a method and system for safe delivery of a drug by infusion, wherein the identity of the drug in a drug container set up for the infusion is verified, and the concentration of the drug is repeatedly measured during the infusion and the infusion rate adjusted when necessary to maintain a desired or prescribed average dose rate for the drug. Such a system for intravenous infusion is schematically illustrated in Fig. 1.

A drug container, here exemplified by a bag 1 or a syringe 2, is connected to a flow pump 3, which when the container is an infusion bag typically may be a peristaltic pump, and in case of a syringe, a syringe pump.

The pump 3 is via a first tubing part 4 connected to a detection chamber, here not visible being concealed by a surrounding analytical detection unit 5 for non-invasive detection of drug solution. The latter is in turn via a second tubing part 6 containing a control valve 7 connected to a patient 8 through e.g. a hypodermic needle or peripheral cannula (not shown).

While the detection chamber provided between the tubing parts 4 and 6, depending on the detection system used, may be a defined portion of a single tubing comprising the parts 4 and 6, the chamber typically has a defined width and is optically transparent to provide an "optical window" for spectroscopic analysis, e.g. of the "closed cuvette" type. Such analysis may be based on measuring radiation that is transmitted, adsorbed or reflected by the fluid in the detection chamber, depending on the type of spectroscopic system. Optionally, the detection chamber may be arranged in the flow pump 3, e.g. as described in the aforementioned WO 2004/033003 A1.

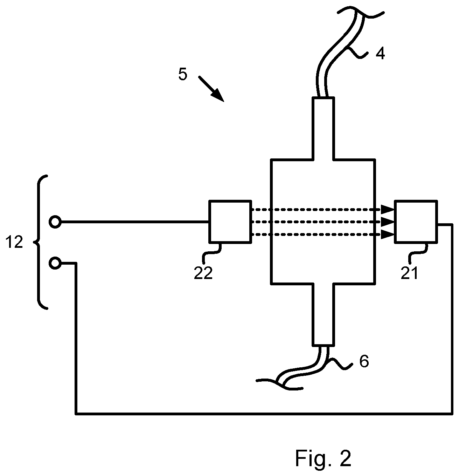

Now with reference made to Fig. 2 a preferred analytical detection unit 5 will be disclosed. This preferred analytical detection unit 5 comprises a spectrophotometric measuring setup, comprising a light source 21 and a photodetector. The light source 22 is configured to transmit light through the detection chamber, here designated 23. The photodetector is configured to receive the light transmitted through the detection chamber and to send a signal comprising light absorbance for a plurality of wavelengths. A preferred spectrophotometric setup provides 1024 measuring channels i.e. bins that the wavelength region of interest is mapped onto. This way the signal contains detailed absorbance data for each bin that forms the wavelength spectra of interest.

Now with reference made to Fig. 1 again, the detection chamber comprises a short cylinder of quartz or UV-transparent plastic, such as Topas^{™} (a cyclic olefin polymer) with Luer fittings cooperating with Luer fittings on the tubing parts 4 and 6.

Exemplary analytical techniques permitting measurements of both the identity and concentration of a drug component in an infusion liquid include fluorescence spectrophotometry and Raman spectroscopy. Optionally, the analytical detection unit includes dual measurement devices, one for the molecular verification and one for the concentration measurements. Analytical techniques for molecular verification additionally include, e.g. NMR and ESR. For a more detailed description of optical measurements in infusion devices it may be referred to the aforementioned WO 2004/109262 A1 and WO 2004/033003 A1.

The pump 3 is connected to a control unit (or controller) 10, typically a computer comprising a memory 19 and a processor 20, through a line 11. To the control unit 10 are further connected the analytical detection unit 5 through a transfer line 12, and the control valve 7 through a line 13. In the illustrated case, the control unit 10 is also connected to a calibration data storage (or memory) 14, which may e.g. also contain patient treatment protocol as well as other data, through a line 15, and to an operation control display 16 and a printer 17, respectively, through a line 18.

In one preferred embodiment the calibration data storage 14 comprises reference spectrophotometric curves for drugs of interest. This data is available to the processor through line 15. The calibration data storage may be a remote server or a cloud service available through internet. In other embodiments it may be a hard disc, or a removable memory such as a USB thumb drive, connected to the control unit 10.

Indicated by dashed contours are two additional sets of drug container, pump and analytical detection unit connected to the patient and the control unit via dotted tubing parts and lines, identical components being indicated by primed and double-primed numerals, respectively.

While the drug, or pharmaceutical, may be any drug given to a patient through infusion, an important application of the present invention is the verification of drug liquids used for chemotherapy of cancer, where the prescription is tailored for each patient and any errors in the type of drug and its concentration may have very serious and even lethal effects on the patient.

An appropriate order for intravenous infusion to a specific patient typically specifies the dose of the drug in question to be given over a specific interval and the concentration of the drug in solution. A drug container, such as e.g. a bag or syringe, is usually provided with a medication label, indicating (at least) the identity of the drug and the concentration thereof. The necessary flow rate for infusion of drug from the drug container is calculated by the medical staff.

In other embodiments, the dose of the drug may be determined by means of a sensor device configured to measure a parameter of the patient, such as for example temperature, pulse, EKG, ECG or cell activity, wherein the sensor device may be connected to the control unit for automatic control of the drug dose. This way a more optimum dose may be infused into the patient.

The required dose rate for the drug is first calculated by dividing the dose, e.g. in µg, by the time interval, e.g. in minutes, to obtain the dose rate R in e.g. µg/min. To calculate the infusion rate (flow rate), the dose rate R (µg/min) is then divided by the concentration C, e.g. in µg/mL, to obtain the infusion rate as R/C in mL/min.

Sometimes it is desired or prescribed that the delivered dose rate should vary over time according to a predetermined protocol which is programmed in the control unit 10. For example, the infusion time may be divided into two or more intervals, each interval having a different (fixed) infusion rate to obtain a corresponding predetermined or prescribed dose rate.

When using the drug delivery system illustrated in Fig. 1, the calculated infusion rate is set on the pump 3 and the infusion is started. As liquid from the drug container 1 or 2 passes through the detection chamber the identity of the drug and its concentration are analyzed by the analytical detection unit 5 in conjunction with the control unit 10. For example, in a preferred embodiment, the analysis may be based on absorption spectrophotometry (IR, visible or UV range), and the optical spectra detected by the detection unit 5 are transferred to the control unit 10 which compares the spectra, i.e. chemical profile, with known data stored in the data storage 14. When the determined chemical profile is verified, the concentration of the drug is calculated from the magnitude of the spectral data transferred from the detection unit 5. If, on the other hand, the drug is not verified by the system, the control unit 10 immediately stops infusion by actuating the control valve 7. Provided that the drug has been verified to be that on the label of the drug container and that the drug concentration corresponds to the label concentration, the infusion of drug solution to the patient is allowed to proceed.

However, if the determined drug concentration deviates from the label concentration, the infusion of drug solution to the patient is still allowed to proceed, but the infusion rate is recalculated by the control unit 10 to a value maintaining the predetermined dose rate for the patient so that the predetermined drug dose has been obtained after the predetermined infusion time length. This recalculated infusion rate is set on the pump 3 by the control unit 10 via the control or feedback line 11.

The operations by the detection unit 5 are continuously repeated during the infusion process at suitable intervals, for example each 10, 15 or 30 seconds, but higher or lower detection frequencies may also be used, as found appropriate. For each detection cycle, a new dose rate calculation is performed based on the instantly measured concentration as well as the cumulative dose of drug infused up to that instant so that as far as possible the predetermined dose rate is maintained (in average) all the time.

For the case that the determined drug concentration is incorrect and far too high so that the patient would have obtained the total dose only during the first few seconds, the infusion is automatically stopped.

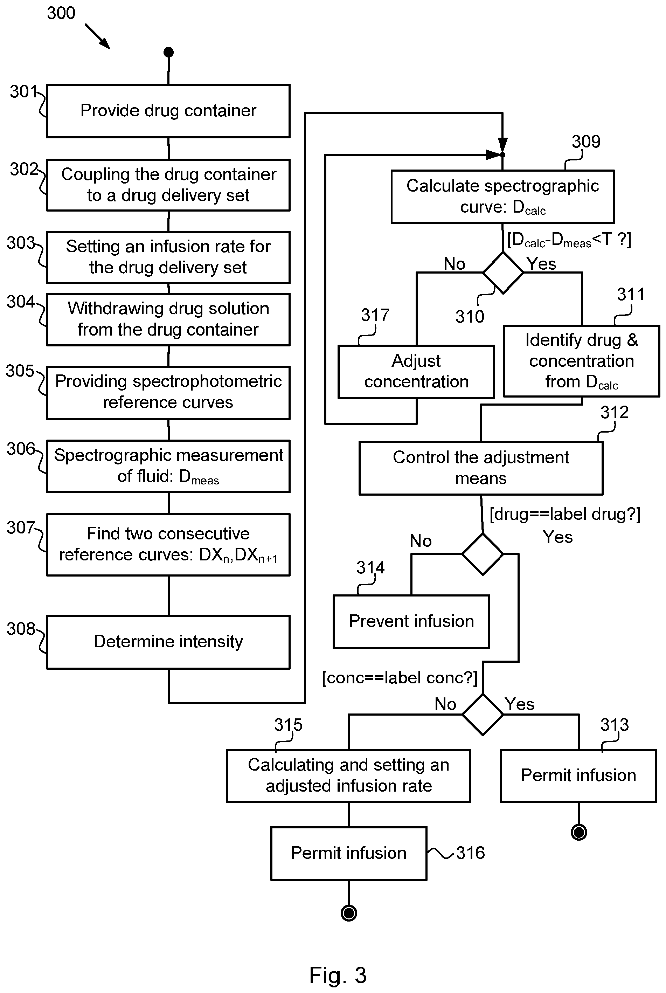

In Fig. 3 a method for administering a drug solution by infusion is disclosed in a flow diagram. The method comprising:
301: Providing a drug container with a label drug solution containing a label concentration of the drug.

302: Coupling the drug container to a drug delivery set for administration of drug solution to a patient by infusion via a fluid path, wherein the fluid path contains a detection chamber,
303: Based on the label concentration of the drug in the drug container, setting an infusion rate for the drug delivery set corresponding to a predetermined dose rate of the drug.

304: Withdrawing drug solution from the drug container through the detection chamber of the analytical detection means,

305: Providing spectrophotometric reference curves with different concentrations for the label drug to be identified. In a preferred embodiment the number of spectrophotometric reference curves is larger than X for each drug, or combination of drugs.

306: Performing a spectrophotometric measurement of the drug solution by means of the analytical detection means, wherein a measured spectrophotometric curve is obtained. The measured spectrophotometric curve comprises a set of wavelength intervals and for each wavelength interval is a corresponding absorbance factor recorded.

307: Finding two consecutive spectrophotometric reference curves from the provided spectrophotometric reference curves, such that the two consecutive spectrophotometric reference curves encompasses the measured spectrophotometric curve of the drug solution in a defined wavelength region. This means that the measured spectrophotometric curve is confined to the region between the two consecutive spectrophotometric reference curves in the defined wavelength region.

308: Determining the intensity for the measured spectrophotometric curve at a first wavelength. In a preferred embodiment the first wavelength corresponds to the wavelength where the difference between the two consecutive spectrophotometric curves is approximatively maximal. This way allows a good resolution in the measured intensity.

309: Calculating a calculated spectrophotometric curve of the fluid by means of the determined intensity using Beer-Lamberts law and the at least two consecutive spectrophotometric reference curves. In one embodiment the determined intensity is used to calculate a concentration of the drug, and the at least two spectrophotometric reference curves are used to determine the parameters in the Beer-Lamberts law. The calculated concentration and the determined parameters are then used in the Beer-Lamberts law for calculating the calculated spectrophotometric curve.

310: Determining if a difference between the calculated spectrophotometric curve and the measured spectrophotometric curve is smaller than a predetermined threshold.

311: Upon determining that the difference is smaller than the predetermined threshold, the drug is identified to be equal to the drug of the two consecutive reference curves, which is the label drug, and a determined concentration of the drug in the fluid is equal to the concentration of the drug in the calculated spectrophotometric curve.

312: Control the adjustment means to calculate and set the adjustment means to an adjusted infusion rate in order to maintain the predetermined dose rate when the drug concentration determined in the detection chamber deviates from the label concentration of the drug.

313: If the label drug is identified, and the determined drug concentration corresponds to the label concentration, permitting infusion of the drug solution at the set infusion rate.

314: If the label drug is not identified, preventing infusion of drug solution.

315: If the label drug is identified but the determined drug concentration does not correspond to the label drug concentration, calculating and setting an adjusted infusion rate for the drug delivery set which at least substantially will maintain the predetermined dose rate of the drug, and permitting infusion of the drug solution at the adjusted infusion rate.

Sometimes two or more drugs may be delivered simultaneously to the patient from e.g. three drug containers, as illustrated by the additional drug containers 1' and 1" and 2' and 2", respectively, which via pumps 3', 3", tubing parts 4', 4", detection units 5', 5" and tubing parts 6', 6" are connected, together with the tubing part 6, to a junction 9 which in turn is connected to the patient needle or cannula (not shown). Other ways of combining the liquid flows from the three tubing parts 6, 6', 6" are, of course, possible, e.g. by combining two tubing parts by a first junction and then adding the third tubing part via second junction.

In such a multiple drug delivery case, the infusion of drug from each drug container is analyzed separately and regulated as described above. While not seemingly feasible or practical today, future analytical methods may be capable of identifying and determining the concentrations of several drugs simultaneously, and it would then be possible to replace the three detection units 5, 5' and 5" by a single analytical detection unit, and the three detection chambers by a single detection chamber passed by the combined flows of drug solutions from the drug containers 1, 1' and 1" or 2, 2' or 2" (or combinations thereof).

In the system described above, flow of drug liquid from an infusion bag could alternatively take place by gravity, omitting the pump 3 and controlling the flow rate through a drip chamber and an adjustable valve which is controlled by the control unit 10. An example of such an automatically regulated drip chamber/valve device is described in US 4,634,426.

In another embodiment, the dose rate of the infused drug may be adjusted in response to a physical parameter of the patient such as temperature, pulse, EKG/ECG information. The physical parameter may be measured with a sensor in contact with the patient and connected to the control unit. This way a closed control loop is formed that may provide an optimum drug infusion to the patient.

In another embodiment, the step of providing a drug container further comprises downloading patient data and a treatment protocol for veryfying the label drug and the label concentration. This way it is possible to verify that the correct drug container is connected to the drug delivery set. It is also possible to receive a desired dos rate profile from a central database containing treatment protocols and procedures.

In yet another embodiment, the method further comprises a step of storing information about the infusion upon completion of infusion. This way patient data can be updated and stored on a database.

As mentioned further above, the whole drug delivery system, except the drug container, could be provided in an infusion pump, for example of multi-channel type to permit simultaneous delivery of multiple drugs.

Optionally, a blood analysis system to measure the biological response to the actual dose rate may be integrated with the drug delivery system, and information obtained therefrom may be used as a variable in the dose rate setting.

The present invention is not limited to the above-described preferred embodiments. Various alternatives, modifications and equivalents may be used. Therefore, the above embodiments should not be taken as limiting the scope of the invention, which is defined by the appending claims.

## Claims

1. A system for administering a drug solution by infusion, comprising:
a drug container (1, 2) containing a label drug solution with a label concentration of a drug,
a drug delivery set comprising a fluid conduit (4, 6) connected to the drug container (1, 2), means for connecting the fluid conduit (4, 6) to a patient (8), a detection chamber in the fluid conduit (4, 6) between the drug container (1, 2) and the patient connection means, and adjustment means for setting an infusion rate corresponding to a predetermined dose rate of the drug based on the label concentration,
analytical detection means (5) for non-invasive determination in the detection chamber of the identity and concentration of the drug in fluid withdrawn from the drug container into to the fluid conduit, and
means (7) for preventing infusion of drug solution from the drug container if the label drug is not identified, and
a control unit (10) arranged to control the adjustment means and configured to calculate and set the adjustment means to an adjusted infusion rate in order to maintain the predetermined dose rate when the drug concentration determined in the detection chamber deviates from the label concentration of the drug, wherein the control unit (10) is configured to perform analysis of the drug concentration sequentially at a plurality of times during the infusion process of the label drug solution from the drug container, and at each time, based on the determined drug concentration and the cumulated infused dose of the drug, calculate a new infusion rate for the drug solution which at least substantially maintains the predetermined dose rate, wherein the analytical detection means uses absorption spectrophotometry for determination of the identity and concentration of the drug in fluid withdrawn from the drug container, whereby optical spectra detected by the analytical detection means (5) are transferred to the control unit (10) which compares the spectra, i.e. chemical profile, with known data stored in a data storage (14).

2. The system according to claim 1, wherein the drug delivery set comprises an infusion pump (3), preferably a peristaltic pump or a syringe pump, and that the pump comprises the adjustment means for setting an infusion rate.

3. The system according to claim 2, wherein the detection chamber, and preferably also the analytical detection means (5) and at least part of the control unit (10), are provided in the infusion pump (3).

4. The system according to any one of claims 1 to 3, wherein the system further comprises a sensor being in contact with the patient and connected to the control unit, wherein the dose rate of the infused drug is adjusted in response to a physical parameter of the patient measured by the sensor.

5. The system according to claim 4, wherein the physical parameter is temperature, pulse or EKG/ECG information.

6. The system according to any one of the preceding claims, wherein said system further comprises a blood analysis system to measure the biological response to the actual dose rate, wherein information obtained therefrom is used as a variable in the dose rate setting.

## Patentansprüche

1. System zum Verabreichen einer Arzneimittellösung durch Infusion, umfassend:
einen Arzneimittelbehälter (1, 2), der eine deklarierte Arzneimittellösung mit einer deklarierten Konzentration eines Arzneimittels enthält,
ein Arzneimittelabgabeset, umfassend eine Fluidleitung (4, 6), die mit dem Arzneimittelbehälter (1, 2) verbunden ist, ein Mittel zum Verbinden der Fluidleitung (4, 6) mit einem Patienten (8), eine Detektionskammer in der Fluidleitung (4, 6) zwischen dem Arzneimittelbehälter (1, 2) und dem Patientenverbindungsmittel, und ein Einstellmittel zum Festlegen einer Infusionsrate, die einer vorbestimmten Dosisrate des Arzneimittels basierend auf der deklarierten Konzentration entspricht,
ein analytisches Detektionsmittel (5) für eine nichtinvasive Bestimmung in der Detektionskammer der Identität und Konzentration des Arzneimittels in Fluid, das aus dem Arzneimittelbehälter in die Fluidleitung entnommen wird und
ein Mittel (7) zum Verhindern der Infusion von Arzneimittellösung aus dem Arzneimittelbehälter, falls das deklarierte Arzneimittel nicht identifiziert ist und
eine Steuereinheit (10), die angeordnet ist, um das Einstellmittel zu steuern, und konfiguriert ist, um eine eingestellte Infusionsrate zu berechnen und das Einstellmittel auf diese festzulegen, um die vorbestimmte Dosisrate aufrechtzuerhalten, wenn die Arzneimittelkonzentration, die in der Detektionskammer bestimmt wird, von der deklarierten Konzentration des Arzneimittels abweicht, wobei die Steuereinheit (10) konfiguriert ist, um eine Analyse der Arzneimittelkonzentration sequenziell zu einer Vielzahl von Zeitpunkten während des Infusionsprozesses der deklarierten Arzneimittellösung aus dem Arzneimittelbehälter durchzuführen und zu jedem Zeitpunkt basierend auf der bestimmten Arzneimittelkonzentration und der kumulierten infundierten Dosis des Arzneimittels eine neue Infusionsrate für die Arzneimittellösung zu berechnen, die die vorbestimmte Dosisrate mindestens im Wesentlichen aufrechterhält, wobei das analytische Detektionsmittel Absorptionsspektrophotometrie für eine Bestimmung der Identität und
Konzentration des Arzneimittels in Fluid verwendet, das aus dem Arzneimittelbehälter entnommen wird, wobei optische Spektren, die durch das analytische Detektionsmittel (5) detektiert werden, an die Steuereinheit (10) übertragen werden, die die Spektren, d. h. das chemische Profil, mit bekannten Daten vergleicht, die in einem Datenspeicher (14) gespeichert sind.

2. System nach Anspruch 1, wobei das Arzneimittelabgabeset eine Infusionspumpe (3), vorzugsweise eine Peristaltikpumpe oder eine Spritzenpumpe, umfasst, und dass die Pumpe das Einstellmittel zum Festlegen einer Infusionsrate umfasst.

3. System nach Anspruch 2, wobei die Detektionskammer und vorzugsweise ebenso das analytische Detektionsmittel (5) und mindestens ein Teil der Steuereinheit (10) in der Infusionspumpe (3) bereitgestellt sind.

4. System nach einem der Ansprüche 1 bis 3, wobei das System ferner einen Sensor umfasst, der in Kontakt mit dem Patienten steht und mit der Steuereinheit verbunden ist, wobei die Dosisrate des infundierten Arzneimittels als Reaktion auf einen physikalischen Parameter des Patienten angepasst wird, der durch den Sensor gemessen wird.

5. System nach Anspruch 4, wobei der physikalische Parameter Temperatur, Puls oder EKG/ECG-Informationen ist.

6. System nach einem der vorstehenden Ansprüche, wobei das System ferner ein Blutanalysesystem umfasst, um die biologische Reaktion auf die tatsächliche Dosisrate zu messen, wobei daraus erhaltene Informationen als eine Variable bei der Dosisratenfestlegung verwendet werden.

## Revendications

1. Système d'administration d'une solution médicamenteuse par perfusion, comprenant :
un contenant de médicament (1, 2) contenant une solution médicamenteuse indiquée sur l'étiquette avec une concentration indiquée sur l'étiquette d'un médicament,
un ensemble d'administration de médicament comprenant un conduit de fluide (4, 6) relié au contenant de médicament (1, 2), des moyens permettant de relier le conduit de fluide (4, 6) à un patient (8), une chambre de détection dans le conduit de fluide (4, 6) entre le contenant de médicament (1, 2) et les moyens de liaison au patient, et un moyen d'ajustement pour définir un débit de perfusion correspondant à un débit de dose prédéterminé du médicament sur la base de la concentration indiquée sur l'étiquette,
un moyen de détection analytique (5) permettant une détermination non invasive dans la chambre de détection de l'identité et de la concentration du médicament dans un fluide prélevé depuis contenant de médicament dans le conduit de fluide, et
un moyen (7) permettant d'empêcher une perfusion de solution médicamenteuse depuis le contenant de médicament si le médicament indiqué sur l'étiquette n'est pas identifié, et
une unité de commande (10) agencée pour commander le moyen d'ajustement et configurée pour calculer et définir le moyen d'ajustement sur un débit de perfusion ajusté afin de maintenir le débit de dose prédéterminé lorsque la concentration de médicament déterminée dans la chambre de détection s'écarte de la concentration indiquée sur l'étiquette du médicament, dans lequel l'unité de commande (10) est configurée pour réaliser une analyse de la concentration de médicament de manière séquentielle à une pluralité de moments pendant le processus de perfusion de la solution médicamenteuse indiquée sur l'étiquette depuis le contenant de médicament, et à chaque fois, sur la base de la concentration de médicament déterminée et de la dose perfusée cumulée du médicament, calculer un nouveau débit de perfusion pour la solution médicamenteuse qui maintient au moins sensiblement le débit de dose prédéterminé, dans lequel le moyen de détection analytique utilise une spectrophotométrie d'absorption pour une détermination de l'identité et de la concentration du médicament dans un fluide prélevé depuis le contenant de médicament, moyennant quoi des spectres optiques détectés par le moyen de détection analytique (5) sont transférés à l'unité de commande (10) qui compare les spectres, c'est-à-dire un profil chimique, avec des données connues stockées dans une unité de stockage de données (14).

2. Système selon la revendication 1, dans lequel l'ensemble d'administration de médicament comprend une pompe à perfusion (3), de préférence une pompe péristaltique ou un pousse-seringue, et le fait que la pompe comprenne le moyen d'ajustement pour définir un débit de perfusion.

3. Système selon la revendication 2, dans lequel la chambre de détection, et de préférence également le moyen de détection analytique (5) et au moins une partie de l'unité de commande (10), sont prévus dans la pompe à perfusion (3).

4. Système selon l'une quelconque des revendications 1 à 3, dans lequel le système comprend en outre un capteur en contact avec le patient et connecté à l'unité de commande, dans lequel le débit de dose du médicament perfusé est ajusté en réponse à un paramètre physique du patient mesuré par le capteur.

5. Système selon la revendication 4, dans lequel le paramètre physique est une température, un pouls ou des informations d'ECG.

6. Système selon l'une quelconque des revendications précédentes, dans lequel ledit système comprend en outre un système d'analyse de sang pour mesurer la réponse biologique au débit de dose réel, dans lequel des informations qui sont obtenues à partir de celui-ci sont utilisées comme variable dans la définition de débit de dose.
